(19) Europäisches Patentamt / European Patent Office / Office européen des brevets

(11) **EP 0 731 173 B1**

(12) **EUROPÄISCHE PATENTSCHRIFT**

(45) Veröffentlichungstag und Bekanntmachung des Hinweises auf die Patenterteilung:
**25.06.2008 Patentblatt 2008/26**

(51) Int Cl.:
*C12Q 1/68* (2006.01)     *G01N 27/447* (2006.01)

(21) Anmeldenummer: **95119546.0**

(22) Anmeldetag: **12.12.1995**

(54) **Verfahren zum direkten Nachweisen weniger Nukleinsäurestränge**

Method for the direct detection of a few oligonucleotides

Procédé pour la détection directe d'un nombre réduit de oligonucléotides

(84) Benannte Vertragsstaaten:
**CH DE FR GB IT LI**

(30) Priorität: **10.03.1995 DE 19508366**

(43) Veröffentlichungstag der Anmeldung:
**11.09.1996 Patentblatt 1996/37**

(73) Patentinhaber: **Evotec AG**
**22525 Hamburg (DE)**

(72) Erfinder:
- **Eigen, Manfred, Prof. Dr.**
  **c/o Max-Planc-Institut für biophysikalische Chemie**
  **37077 Göttingen (DE)**
- **Rigler, Rudolf, Dr.**
  **St. Sulpice, CH-1025 (CH)**

(74) Vertreter: **Meyers, Hans-Wilhelm et al**
**Patentanwälte**
**von Kreisler Selting Werner**
**Bahnhofsvorplatz 1**
**Deichmannhaus am Dom**
**50667 Köln (DE)**

(56) Entgegenhaltungen:
WO-A-90/04652     WO-A-93/20236
WO-A-94/16313     WO-A-95/19449

- EIGEN M ET AL: "SORTING SINGLE MOLECULES: APPLICATION TO DIAGNOSTICS AND EVOLUTIONARY BIOTECHNOLOGY" PROCEEDINGS OF THE NATIONAL ACADEMY OF SCIENCES OF USA, US, NATIONAL ACADEMY OF SCIENCE. WASHINGTON, Bd. 91, Juni 1994 (1994-06), Seite 5740-5747 XP002029412 ISSN: 0027-8424
- PATENT ABSTRACTS OF JAPAN vol. 017, no. 462 (C-1101), 24. August 1993 (1993-08-24) & JP 05 111399 A (HITACHI LTD), 7. Mai 1993 (1993-05-07)
- RIGLER R ET AL: "FLUORESCENCE CORRELATION SPECTROSCOPY AND APPLICATION TO THE STUDY OF BROWNIAN MOTION OF BIOPOLYMERS" PHYSICA SCRIPTA, SE, STOCKHOLM, Bd. 19, 1979, Seite 486-490 XP000195642
- RIGLER R: "FLUORESCENCE CORRELATIONS, SINGLE MOLECULE DETECTION AND LARGE NUMBER SCREENING APPLICATIONS IN BIOTECHNOLOGY" JOURNAL OF BIOTECHNOLOGY, NL, ELSEVIER SCIENCE PUBLISHERS, AMSTERDAM, Bd. 41, Nr. 2/03, Juli 1995 (1995-07), Seite 177-186 XP000670350 ISSN: 0168-1656

## Beschreibung

Die vorliegende Erfindung betrifft ein Verfahren zum direkten Nachweisen weniger, vorzugsweise einzelner Nucleinsäurestränge einer bestimmten Zielsequenz in einer Untersuchungslösung.

Die Erfindung betrifft ferner eine Vorrichtung zur Durchführung des Verfahrens sowie eine dabei verwendete Testlösung.

Es ist allgemein wünschenswert, DNA/RNA-Moleküle bekannter, teilweise bekannter und/oder auch unbekannter Sequenz nachweisen zu können. Anwendungsbeispiele hierfür sind genetische Untersuchungen, Analysen von Laborexperimenten z.B. im Rahmen der evolutiven Biotechnologie oder vor allem auch die Virusdiagnostik. In all diesen Anwendungsfällen befinden sich in der ursprünglich vorliegenden Untersuchungslösung häufig nur wenige oder gar einzelne Nucleinsäurestränge der bestimmten Zielsequenz, die in der Untersuchungslösung vorzugsweise quantitativ nachgewiesen werden sollen.

Zur Zeit erfolgt der Nachweis einer Virusinfektion im allgemeinen auf indirektem Wege, nämlich über die Immunantwort des befallenen Wirtes. Der Test ist mit einer Reihe von Schwierigkeiten verbunden, die diesem indirekten Verfahren anhaften. Dazu zählen die lange Latenzzeit der verzögerten Immunantwort, die zeitraubende Analyse z.B. eines Elisa-Testes, sowie die Möglichkeit einer falsch-positiven bzw. falsch-negativen Immunantwort.

Es besteht daher das Bestreben, einen direkten Test zu erarbeiten, bei dem das Virus hochspezifisch nachgewiesen werden kann. Die Schwierigkeiten eines direkten Nachweises liegen vor allem in der geforderten hohen Empfindlichkeit. Im Grenzfall muß es nämlich möglich sein, ein einziges Viruspartikel in einem Zellextrakt bzw. in einem Serum nachweisen zu können. Die Konzentration der nachzuweisenden Nucleinsäurestränge liegt dabei im Bereich zwischen $10^{-12}$ und $10^{-18}$ molar.

Ein vor einigen Jahren entwickeltes Verfahren besteht darin, die einzelnen oder wenigen Nucleinsäurestränge auf ein Konzentrationsniveau hochzuverstärken, das konventionellen Methoden wie z.B. der Gelelektrophorese, dem Verfahren der Temperaturgradientengele, der Sequenzierung bspw. nach dem Sanger-Verfahren oder der Maxam-Gilbert-Technik zugänglich ist. Für dieses Hochverstärken stehen die Polymerase-Kettenreaktion (PCR) oder analoge Verfahren zur Verfügung, die auf einer spezifischen Replikation des nachzuweisenden Nucleinsäureabschnittes beruhen. Dabei nutzt man die Eigenschaften von DNA-Polymerasen aus, die einen Einzelstrang zum Doppelstrang aufpolymerisieren können, sofern ihnen ein kurzer, doppelsträngiger Bereich als Primer zur Verfügung steht. Dabei werden die Nucleinsäurestränge, die eine zu amplifizierende Sequenz enthalten, mit einem Überschuß von zwei chemisch synthetisierten Oligonucleotiden versetzt, die aus den Randbereichen der zu amplifizierenden Sequenz stammen und strangspezifisch sind, d.h. komplementär zu einem der beiden DNA-Stränge.

Zum einen sind derartige Verfahren sehr zeitaufwendig, es ist nicht nur erforderlich, die PCR-Reaktion durchzuführen, danach müssen vielmehr weitere Analysen vorgenommen werden, um zumindest qualitativ auf die Anwesenheit der gesuchten Sequenz in der Untersuchungslösung schließen zu können. Ferner ist es hier erforderlich, daß bestimmte kurzkettige Nucleinsäuresequenzen mit einer Länge von etwa 15 - 20 Nucleotiden bereitgestellt werden müssen, die exakt komplementär zu einem entsprechenden Abschnitt der gesuchten Sequenz sind. Man nennt einen solchen exakt komplementären kurzkettigen Nucleinsäureabschnitt allgemein "Antisense-Sequenz" oder auch "Primer".

Neben dem Zeitaufwand ist bei diesem Verfahren weiter von Nachteil, daß nur Nucleinsäuresequenzen nachgewiesen werden können, bei denen zumindest ein kurzer Abschnitt der Sequenzabfolgen an den beiden Enden bekannt ist, so daß die entsprechenden Primer bereitgestellt werden können. Handelt es sich bei den nachzuweisenden Nucleinsäuresträngen um RNA-Moleküle, so müssen diese zunächst sogar noch mittels einer RNA-abhängigen DNA-Polymerase in eine DNA-Kopie umgeschrieben werden, bevor die PCR-Technik eingesetzt werden kann.

Wegen der ggf. vielen verschiedenen Verfahrensschritte weist diese Methode viele Fehlerquellen auf, da die einzelnen Polymerasen nur mit einer bestimmten Wahrscheinlichkeit die "richtigen" komplementären Nucleotide bei der Polymerisation einbauen, so daß auch hier nicht ausgeschlossen werden kann, daß es zu falsch-positiven und falsch-negativen Testergebnissen kommt.

Ein weiteres Verfahren besteht darin, die gesuchte Sequenz direkt sichtbar zu machen, was bedeutet, daß sie sich zunächst natürlich eindeutig von alternativen Sequenzen unterscheiden muß. Ein neu entwickeltes Verfahren, um einzelne Moleküle zu identifizieren und zu zählen, ist die in der WO 94/16313 beschriebene Korrelationsfluoreszenz-Spektroskopie, die im folgenden kurz FCS genannt wird. Die genannte Druckschrift beschreibt, daß mittels der FCS in einem sehr kleinen Meßvolumen im Bereich von 0,1 - 10 fl einzelne mit Farbstoff markierte DNA/RNA-Moleküle nachgewiesen werden können, sofern sich das nachzuweisende Molekül entweder deutlich von alternativen Molekülen im Meßvolumen unterscheidet oder aber isoliert vorliegt.

Bei der bereits beschriebenen PCR-Methode benötigt man zwei Primersequenzen, von denen die eine komplementär zum Stranganfang des Plusstranges, die andere komplementär zum Anfang des Minusstranges ist. Der Sequenzabschnitt zwischen den Positionen der beiden Primer-Sequenzen wird dann verstärkt. Beim FCS-Nachweis dagegen benötigt man nur eine spezifische Primer-Sequenz, die mit eimem Fluoreszenzfarbstoff markiert ist. Sie lagert sich direkt an die zu detektierende (einzelsträngige) RNA-Sequenz oder an einen Strang der aufgeschmolzenen DNA-Sequenz an. In dieser Form unterscheiden sich die markierten Primer sowohl in ihren Ladungseigenschaften als auch in ihrer Beweglichkeit sehr stark von den ungebundenen Primern und können ohne weitere Verstärkung durch die FCS direkt detektiert werden.

Das Meßprinzip der FCS beruht darauf, daß fluorophore Moleküle in äußerst verdünnten Lösungen gemessen werden, indem ein relativ kleines Volumenelement der Lösung einem starken Anregungslicht eines Lasers ausgesetzt wird. Nur die Moleküle entsprechenden Anregungsspektrums, die sich in diesem Meßvolumen aufhalten, werden durch das Licht angeregt. Das emittierte Fluoreszenzlicht aus diesem Volumenelement wird dann auf einen Photomultiplier abgebildet. Handelt es sich um verdünnte Lösungen, so ergeben sich nennenswerte Schwankungen der Konzentration der sich in dem jeweiligen Volumenelement befindlichen Moleküle.

Ein Molekül, das einmal in das Volumenelement hineindiffundiert ist, wird sich gemäß seiner charakteristischen Diffusionsgeschwindigkeit in einer durchschnittlichen, aber für das betreffende Molekül charakteristischen Zeit wieder aus dem Volumenelement entfernen und dann nicht mehr zu beobachten sein.

Wird nun die Lumineszenz ein und desselben Moleküles während seiner durchschnittlichen Aufenthaltsdauer in dem Meßvolumen viele Male angeregt, so lassen sich von diesem Molekül folglich viele Signale erfassen. Bei verdünnten Lösungen ist damit die Wahrscheinlichkeit, daß ein einmal in das Meßvolumen hineindiffundiertes Molekül vor seinem Wiederaustritt nochmals angeregt wird, viel größer, als dies für ein neu eintreffendes Molekül der Fall ist.

In der oben erwähnten WO 94/16313 wird ausgeführt, daß es auf der Basis dieses Meßprinzipes möglich ist, einzelne Moleküle in verdünnten Lösungen zu erfassen. Handelt es sich bei den nachzuweisenden Molekülen um Nucleinsäurestränge, so wird ein zu einer bestimmten Teilsequenz des nachzuweisenden Moleküles komplementärer farbstoffmarkierter Primer zugegeben, dessen Diffusionsverhalten im ungebundenen Zustand sich von dem im an das Zielmolekül gebundenen Zustand unterscheidet.

Um die von der Diffusionsgeschwindigkeit der beteiligten Moleküle abhängige z.T. relativ lange Meßzeit zu reduzieren, beschreibt die erwähnte Druckschrift verschiedene Verfahren, mit denen die nachzuweisenden Moleküle im Meßvolumen aufkonzentriert werden können. Im Prinzip beruhen diese Verfahren darauf, die unterschiedlichen Ladungen von ungebundenen Primern und Primer-Nucleinsäurestrang-Komplexen auszunutzen, um in einem gerichteten elektrischen Feld entweder die ungebundenen Primer von den Komplexen zu trennen, oder aber die unterschiedliche Driftgeschwindigkeit auszunutzen.

Ein anderes Verfahren, um die Spezifität des Nachweises zu erhöhen, besteht darin, zwei verschiedene Primer zu benutzen, die unterschiedlich markiert sind. Durch eine zeitliche Korrelation der von den verschieden markierten Primern stammenden Fluoreszenzsignale lassen sich im Wege der elektronischen Signalverarbeitung dann die Signale der nicht-korrelierten, also freien Primer effizient unterdrücken. Ein derartiges Verfahren ist auch aus Eigen H. et al (Proc. Natl. Acad. Sci. USA, Vol 91, 5740-5747, Juni 1994) bekannt.

Weiter wird beschrieben, daß sich durch ein niederfrequentes elektrisches Wechselfeld von z.B. 4 Hz eine Oszillation der geladenen Moleküle durch das Meßvolumen erzwingen läßt, wodurch die Moleküle quasi fokussiert werden, so daß es möglich ist, nur ein einziges Molekül oder wenige Moleküle im Meßvolumen quantitativ zu erfassen.

Die erwähnte Druckschrift beschreibt viele Anwendungsmöglichkeiten der FCS, beschäftigt sich aber genauer nur mit der Vorrichtung, der Optik und den verschiedenen Molekülfallen. Allerdings wird unter anderem erwännt, daß mit der FCS-Apparatur auch einzelne DNA/RNA-Stränge unter Verwendung von Primern gezählt werden können. Großes Augenmerk richtet die PCT-Anmeldung auch auf die Messung physikalischer Parameter, wozu u.a. unterschiedlich markierte Liganden eingesetzt werden, was jedoch nicht zur Verbesserung der Intensität der Meßsignale, sondern zur Erhöhung der Spezifität erfolgt. Bei derartig unterschiedlich markierten Sonden wird nämlich eine Kreuzkorrelationstechnik angewendet, um weitere Parameter wie z.B. die Rotationsgeschwindigkeit des nachzuweisenden Moleküles bestimmen zu können.

Allgemein wird beschrieben, daß elektrophoretische Trennverfahren, Kapillaren, Gleichfelder etc. verwendet werden können, um der Diffusionsgeschwindigkeit der beteiligten Moleküle eine Driftgeschwindigkeit zu überlagern. Die Primer und die Zielmoleküle können dabei so ausgewählt werden, daß sie unterschiedliche Ladungen aufweisen und damit im elektrischen Feld unterschiedlich wandern.

Die FCS-Methode weist somit gegenüber dem PCR-Verfahren oder analogen Methoden eine Reihe von Vorteilen auf. Zunächst läßt sich die FCS-Methode sowohl auf einzelsträngige als auch auf doppelsträngige Nucleinsäuremoleküle direkt anwenden. Da viele Pathogene einsträngige RNA-Viren sind, müssen diese bei dem FCS-Verfahren nicht zunächst in DNA umkopiert werden, was sich im Zeitaufwand für die Analyse positiv bemerkbar macht. Darüber hinaus ist die FCS-Methode direkter, sie weist nämlich die einzelnen Nucleinsäuren nach, die im Falle von PCR erst vervielfältigt werden müssen, was ebenfalls einen Unterschied im Zeitaufwand bedingt.

Darüber hinaus kann der Verstärkungsprozeß durch bestimmte Substanzen gestört oder gehemmt werden, so daß das PCR-Verfahren für natürliche Proben nicht immer zuverlässig ist.

Genauso wie das PCR-Verfahren ist die FCS-Methode jedoch in bezug auf die Spezifität störanfällig. Eine Primersequenz kann nämlich z.B. in Teilabschnitten auch zu Sequenzen komplementär sein, die der Zielsequenz zufällig ähnlich sind. Man wählt deshalb Primersequenzen der Länge 15 bis 20 Nucleotide aus, um eine hinreichende Spezifität bei der Bindung zu erzielen. Dann muß aber auch sichergestellt werden, daß die Schmelzpunkte von nur teilweise komplementären Sequenzen unterhalb der Arbeitstemperatur liegen, also meßtechnisch nicht erfaßt werden.

Beim PCR-Verfahren verwendet man zwei Primersequenzen, wobei die Wahrscheinlichkeit, daß beide Sequenzen Fehlbindungen aufweisen, gering ist. Dafür müssen die Primer hier aber in sehr viel höherer Konzentration eingesetzt werden, da sie durch die Reaktion verbraucht werden.

Bei der FCS-Methode muß die Primerkonzentration dagegen lediglich so hoch sein, daß eine Anlagerung an die nachzuweisenden Nucleinsäurestränge innerhalb der Meßzeit auch erfolgen kann.

Diese Bedingungen schränken die Anwendbarkeit der Methode auf kleine Absolutmengen erheblich ein.

Hiervon ausgehend ist es Aufgabe der vorliegenden Erfindung, ein Verfahren von der eingangs genannten Art zu schaffen, das die oben erwähnten Nachteile und Schwierigkeiten überwindet sowie einen schnellen und sicheren direkten Nachweis von Nucleinsäuresträngen ermöglicht.

Erfindungsgemäß wird diese Aufgabe einerseits gelöst durch ein Verfahren zum direkten Nachweisen weniger, vorzugsweise einzelner Nucleinsäurestränge einer bestimmten Zielsequenz (10) in einer Untersuchungslösung, mit den Schritten:

a) Bereitstellen einer Testlösung mit einem Gemisch (11) unterschiedlicher, kurzer Primer (12, 13, 14), die jeweils eine zu einem Abschnitt (a, b, c) der Zielsequenz (10) komplementäre, sogenannte Antisense-Sequenz (a', b', c') aufweisen und mit einem oder mehreren gleichen Fazbstoffmolekülen (16) markiert sind,

b) Mischen der Testlösung mit der Untersuchungslösung und Inkubieren dieser gemischten Lösung (15) unter Hybridisierung der Primer (12, 13, 14) mit den nachzuweisenden Nucleinsäuresträngen zulassenden Randbedingungen, und dann

c) Identifizieren der Zielsequenz (10) in der inkubierten Lösung (15) mit einer Apparatur (20) für optische Analysen mittels zeitaufgelöster Fluoreszenzspektroskopie, die dazu ausgelegt ist, wenige; vorzugsweise einen der nachzuweisenden Nucleinsäurestränge, an die bzw. den mehrere Primer (12, 13, 14) hybridisiert sind, vor dem Hintergrund der nicht hybridisierten Primer (12, 13, 14) zu diskriminieren.

Die der Erfindung zugrundeliegende Aufgabe wird auf diese Weise vollkommen gelöst. Durch die Hybridisierung vieler Primer mit der Zielsequenz wird nämlich die lokale Konzentration der Primer bspw. im Meßvolumen bei der FCS-Technik stark erhöht, wenn eine Zielsequenz mit angelagerten Primern in das Meßvolumen diffundiert, was als "Lichtblitz" im registrierten Fluoreszenzsignal zu sehen ist. Die Erfinder der vorliegenden Anmeldung haben nämlich erkannt, daß es möglich ist, einen derartigen "Cocktail" von Primern zu verwenden, da es bei dem Nachweis einzelner Nucleinsäurestränge lediglich darauf ankommt, das Fluoreszenzsignal zu verstärken. Sequenzen, an die sich quasi "zufällig" ein Primer anlagert, werden wegen des sehr viel geringeren Signales im Gegensatz zu der bekannten FCS-Methode jetzt nicht mehr falsch-positiv angezeigt.

Die Geschwindigkeitskonstante der Hybridisierung zwischen Primern und Zielsequenzen liegt allgemein unterhalb von $10^6\,M^{-1}s^{-1}$, so daß schon bei Primerkonzentrationen von $10^{-9}$ M die Reaktionszeit tausend Sekunden übersteigt. Liegt nun die Zielsequenz selbst nur in wenigen Kopien, z.B. einem Strang pro Mikroliter vor, so wird ein direkter Nachweis unmöglich, denn im genannten Beispiel wäre der ungebundene Primer z.B. in milliardenfachen Überschuß vorhanden. Beiträge der gebundenen Fluoreszenzmarker wären viel zu klein, um nach der FCS-Methode registriert werden zu können.

Dieses Problem wird jedoch erfindungsgemäß dadurch gelöst, daß der Cocktail von Primersequenzen bereitgestellt wird, die zu verschiedenen Regionen des Zielmoleküls komplementär sind. Jede Primersequenz kann im Mittel mit einem Fluoreszenzmarker, die alle aus dem gleichen Fluorophoren bestehen, versehen sein. Dieser Cocktail wird z.B. in einer Konzentration von etwa $10^{-9}$ M angeboten, was ein Molekül pro Femtoliter bedeutet. Bei dem FCS-Verfahren, bei dem die "Lichtfalle" des Laserstrahles ein Volumen von ca. 0,2 fl besitzt, würde also ein "Hintergrundrauschen" registriert, das der Gegenwart von einem Fluoreszenzmarker in etwa 20 % der Zeit entspricht. Gerät nun ein Zielmolekül in die Licht- oder Molekülfalle, so würde das ein plötzliches "Aufleuchten" der Fluoreszenz bewirken, denn nunmehr wären z.B. 20 bis 100 Fluoreszenzmarker in der Lichtfalle gleichzeitig anwesend. Während das markierte Zielmolekül in der mittleren Intensität vollständig untergehen würde, erschiene es in der Fluktuationregistrierung von FCS quasi als "Leuchtbombe".

Prinzipiell ist diese Idee auch auf Tumorzellen anwendbar, um einen schnellen direkten Tumornachweis zu ermöglichen. Hier kann die Tatsache ausgenutzt werden, daß jede Tumorzelle mehrere tausend antigene Determinanten für Antikörper aufweist. Wenn jetzt die Antikörper mit Farbstoffen markiert werden, so kann eine Tumorzelle, an die Antikörper gebunden haben, als "Lichtblitz" bei dem erwähnten FCS-Verfahren nachgewiesen werden. Auch hier wird der Effekt der Aufkonzentration der mit Farbstoff markierten Antikörper durch die Anlagerung an die Tumorzelleç ausgenutzt. Ein auf dieser Idee basierender Tumornachweis wäre eine alternative Anwendung der erfindungsgemäßen Idee, viele verschiedene farbstoffmarkierte Sonden gegen ein Zielobjekt einzusetzen, an dem diese Sonden in größerer Zahl binden können. Hält man die Konzentration der Sonden so gering, daß sie in dem beobachteten Meßvolumen mit einer statistischen Wahrscheinlichkeit auftauchen, die deutlich geringer ist als die Zahl der im Mittel an dem Zielobjekt gebundenen Sonden, so läßt sich die Aufkonzentrierung der Sonden im Meßvolumen messen, wenn ein Zielobjekt mit gebundenen Sonden in das Meßvolumen eintritt.

Andererseits wird diese Aufgabe bei einem Verfahren der eingangs genannten Art gelöst durch die Schritte:

1) Hybridisieren zumindest einiger der nachzuweisenden Nucleinsäurestränge mit jeweils einem langen Antisehse-Strang (17), der mit mehren gleichen Farbstffmoleülen (16) markiert und zu der gesamten oder nahezu der gesamten Zielsequenz (10) komplementär ist, und

2) Identifizieren der Zielsequenz (10) mit einer Apparatur (20) für optische Analysen mittels zeitaufgelöster Fluoreszenzspektroskopie, die dazu ausgelegt ist, wenige, vorzugsweise einen der nachzuweisenden Nucleinsäurestränge, an die bzw. den ein Antisense-Strang (17) hybridisiert ist, zu diskriminieren.

Auch hierdurch wird die der Erfindung zugrundeliegende Aufgabe vollkommen gelöst. Die Erhöhung des Fluoreszenzsignales erfolgt jetzt dadurch, daß der Antisense-Strang mit sehr vielen Farbmarkiert ist, so daß ein derartiger Doppelstrang ebenfalls als "Lichtblitz" im registrierten Fluoreszenzsignal zu sehen ist.

Die Hybridisierung zwischen nachzuweisendem Nucleinsäurestrang und Antisense-Strang kann entweder dadurch erfolgen, daß der Antisense-Strang gesondert hergestellt und wie bei dem Verfahren mit den kurzen Primern zu der Untersuchungslösung hinzugegeben sowie einem geeigneten Temperverfahren unterzogen wird.

Andererseits ist es auch möglich, den farbstoffmarkierten Doppelstrang aus nachzuweisendem Nucleinsäurestrang und Antisense-Strang durch direktes Aufpolymerisieren des nachzuweisenden Nucleinsäurestranges zu erzeugen, wobei farbstoffmarkierte Nucleinsäure-(oder analoge) Bausteine eingesetzt werden. Vorzugsweise verwendet man hier markierten UTPs. Sollte sich der so erzeugte Doppelstrang, der mit sehr vielen Farbstoffmolekülen markiert ist, vor dem Hintergrund der freien, ebenfalls markierten Bausteine nicht nachweisen lassen, so müssen geeignete Trennverfahren vorgeschaltet werden, bevor die den markierten Doppelstrang enthaltende Lösung bspw. in eine FCS-Apparatur gegeben wird, wie sie weiter unten noch ausführlicher beschrieben ist.

In einem Ausführungsbeispiel des ersten erfindungsgemäßen Verfahrens werden in Schritt a) zu unterschiedlichen, vorzugsweise nicht überlappenden Abschnitten der Zielsequenz komplementäre Primersequenzen bereitgestellt, wobei unterschiedliche Primersequenzen sich in der Sequenzabfolge der Nucleinsäure- (oder analoger) Bausteine voneinander unterscheiden.

Hier ist von Vorteil, daß die Zahl der an eine Zielsequenz bindenden Primersequenzen besonders groß ist, so daß eine hohe Aufkonzentrierung der Primersequenzen im Meßvolumen erfolgt, was eine größere Sicherheit gegen falsch-positive Meßergebnisse wegen zufällig passender einzelner Primer gewährleistet.

Dabei ist es dann bevorzugt, wenn in Schritt a) Primer unterschiedlicher Frequenzlänge, also mit unterschiedlicher Zahl von Nucleinsäure- (oder analoger) Bausteinen pro Primer bereitgestellt werden, wobei die Primer eine Sequenzlänge zwischen 10 und 50, vorzugsweise 15 und 20 Bausteinen aufweisen.

Durch die unterschiedlichen Primerlängen kann in vorteilhafter Weise den Sekundärstrukturen der nachzuweisenden Nucleinsäurestränge Rechung getragen werden. Kurzsträngige Primer würden dabei gegen einzelsträngig vorliegende Bereiche der Nucleinsäurestränge eingesetzt werden, während längere Primersequenzen z.B. gegen doppelsträngig vorliegende Bereiche eingesetzt würden, die zunächst durch entsprechende Temperaturwahl aufgeschmolzen werden müßten, wobei die Primer dann in Konkurrenz zu der Ausbildung der Sekundärstruktur treten würden. Wegen der größeren Anzahl von Bausteinen ist die Bindung zwischen längerkettigen Primern und den zugeordneten Bereichen der Zielsequenz hier so groß, daß die Primerbindung vorrangig vor der erneuten Ausbildung der Sekundärstruktur erfolgt. Es hat sich herausgestellt, daß Primer mit einer Sequenzlänge zwischen 15 und 20 Bausteinen einerseits eine hinreichend spezifische Bindung mit komplementären Bereichen der Zielsequenz eingehen und andererseits keine zufälligen unspezifischen Bindungen zulassen.

In einer Weiterbildung ist es dann bevorzugt, wenn Primer mit 10 bis 200, vorzugsweise 20 bis 100 verschiedenen Sequenzen bereitgestellt werden.

Auf diese Weise würden zwischen 300 und 2.000 komplementäre Bausteine an die Zielsequenzen angelagert werden, die als Virus-RNA bspw. eine Sequenzlänge zwischen 4.000 und 10.000 Bausteinen aufweist, so daß sie bei einer derartigen Hybridisierung im Mittel die maximale Anzahl von anzulagernden Primern aufnimmt. Von Vorteil ist dabei, daß es zu einer sehr starken Aufkonzentrierung an Primern und damit einer starken Erhöhung des fluktuierenden Fluoreszenzsignales kommt, wenn ein derartiger Komplex aus Zielmolekül und Primern in das Meßvolumen wandert, was die Meßsicherheit weiter erhöht.

Allgemein ist es bevorzugt, wenn die Primer durch direkte Synthese, bspw. mittels eines Nucleinsäure-Synthetisierers hergestellt werden.

Hier ist von Vorteil, daß ein absolut sicherer Weg zur Bereitstellung der verschiedenen Oligonucleotide (Primer) beschritten wird. Dieser Weg ist zwar zeitraubend, schließt aber eine mögliche Präsenz der gesamten Zielsequenz innerhalb des angebotenen "Cocktails" und damit eine Fehlanzeige der Zielsequenz aus. Ferner lassen sich überlappende Primersequenzen vermeiden, wodurch eine gezielte Markierung möglich ist. Allerdings muß die Zielsequenz genau bekannt sein.

Andererseits ist es bevorzugt, wenn die Primer durch Replikation oder Transkription der Zielsequenz in Gegenwart von mit Farbstoffmolekülen markierten Nucleinsäure- (oder analogen) Bausteinen hergestellt werden, wobei die Replikations- oder Transkriptionsprodukte anschließend in Teilsequenzen zerschnitten werden, die dann als Primer verwendet werden.

Dieses Verfahren hat den Vorteil, daß es deutlich schneller abläuft, allerdings enthält der Cocktail zu Beginn des Verfahrens noch die Zielsequenz, die jedoch nicht genau bekannt sein muß. Es muß also sichergestellt werden, daß bei dem Zerschneiden in jedem Falle die Zielsequenz abgebaut wird, um eine Fehlangabe zu verhindern. Dieses Zerschneiden der Replikations- oder Transkriptionsprodukte kann bspw. auf mechanischem Wege erfolgen, indem Scherkräfte aufgewandt werden, die die Produkte zerschlagen.

Andererseits ist es möglich, die Replikations- oder Transkriptionsprodukte für bestimmte Zeitspannen der Wirkung spezifisch und/oder unspezifisch schneidender Nucleasen auszusetzen, um sie zu Primern abzudauen.

Hier ist von Vorteil, daß dieser Abdau zeitlich so gesteuert werden kann, daß Primer mit einer gewünschten mittleren Länge entstehen.

Insgesamt ist es bevorzugt, wenn die Replikations- oder Transkriptionsprodukte durch Verwendung der Polymerase-Kettenreaktionstechnik, eines analogen Verfahrens oder mit Hilfe anderer spezifischer RNA- bzw. DNA-Polymerasen hergestellt werden.

Hier handelt es sich um gut eingeführte Verfahren, die in vorteilhafter Weise angewendet werden können, um aus der Zielsequenz die Primer zu erzeugen. In vorteilhafter Weise können hier Primer gegen Zielsequenzen hergestellt werden, deren Sequenzabfolge nur teilweise oder gar nicht bekannt ist. Es ist lediglich erforderlich, daß die Enden der Zielsequenzen bekannt sind oder daß bekannte Sequenzabschnitte an völlig unbekannte Zielsequenzen ansynthetisiert werden, so daß die für das PCR-Verfahren erforderlichen Primer bereitgestellt werden können. Der zwischen den beiden Primern liegende Bereich der Zielsequenz muß nicht zwingend in der Abfolge bekannt sein, die Primer für die Analyse werden sozusagen "automatisch" richtig erzeugt, so daß z.B. auch neue Viren gesucht werden können.

Weiter ist es bevorzugt, wenn mittels eines DNA-Synthesizers kurze DNA-Stränge hergestellt werden, die Abschnitte der Zielsequenz aufweisen, wenn diese DNA-Stränge dann bspw. mit der T7-Polymerase transkribiert und die Transkriptionsprodukte als Primer verwendet werden.

Hier ist von Vorteil, daß auf bekanntem Wege RNA-Primer hergestellt werden können, die dann erfindungsgemäß als Cocktail gegen eine nachzuweisende RNA-Zielsequenz eingesetzt werden können, ohne daß dazu die gesamte Sequenzabfolge der Zielsequenz bekannt sein muß.

Allgemein ist es bevorzugt, wenn als Primer DNA-, RNA- oder PNA-Sequenzen verwendet werden.

Hier ist von Vorteil, daß DNA- und RNA-Zielsequenzen nachgewiesen werden können, wobei die Verwendung von ggf. positv geladenen PNA-Sequenzen, die ein Peptid-ähnliches Rückgrat aufweisen und daher nicht - wie RNA und DNA - negativ geladen sind, eine elektrophoretische Trennung zwischen freien und komplexierten Primern ermöglicht. Durch Anhängen einer positiven Restgruppe können PNAs auch mit positiver Ladung versehen werden, was die Trennung weiter erleichtert.

Weiter ist es bevorzugt, wenn in Schritt b) die gemischte Lösung bei einer Temperatur inkubiert wird, die so hoch ist, daß Tertiär- und Sekundärstrukturen der Zielsequenz aufschmelzen, die hinreichend tief ist, so daß die spezifischen Bindungen zwischen Primer und Zielsequenz nicht aufschmelzen, und die hinreichend hoch ist, so daß unspezifische Bindungen zwischen Primer und Zielsequenz aufschmelzen.

Hierdurch wird in vorteilhafter Weise ein wichtiges Problem bei der Markierung gelöst. Die Geschwindigkeitskonstante für die Anlagerung von Primersequenzen liegt nämlich im allgemeinen unterhalb von $10^6$ $M^{-1}s^{-1}$, so daß bei den vorteilhafterweise eingesetzten Primerkonzentrationen von $10^{-9}$ $M^{-9}$ bereits Reaktionszeiten von mehr als 1.000 Sekunden auftreten. Diese Werte sind nun aber stark temperaturabhängig, denn die Sekundärstruktur der Zielsequenz muß aufschmelzen, bevor die Primersequenzen sich anlagern können. Andererseits muß man aber bei Temperaturen arbeiten, die unterhalb des Schmelzpunktes der Primerbindung liegen, wobei zur Ausschaltung von unspezifischen Fehlanlagerungen die Temperatur direkt unterhalb dieses Schmelzpunktes liegen sollte.

Aus all dem folgt, daß die Markierung der Zielsequenzen mit dem Primer-Cocktail vorteilhafterweise durch einen genau festgelegten Temperprozeß zu erzielen ist, der die genannten Schmelzpunkte quantitativ berücksichtigt.

In einer Weiterbildung ist es hier bevorzugt, wenn die Primer hierzu in großem Überschuß gegenüber der Zielsequenz eingesetzt werden, wobei nach erfolgter Inkubation die ungebundenen Primer von der hybridisierten Zielsequenz abgetrennt werden können.

Hier ist von Vorteil, daß man zunächst bei höheren Primerkonzentrationen eine Sättigung der Hybridisierung vornehmen und anschließend die unreagierten Primer abtrennen kann.

Dies ist insbesondere dann auf einfachem elektrophoretischem Wege möglich, wenn als Primer positiv geladene PNA-Sequenzen verwendet werden.

Das Identifizieren der mit den Primern hybridisierten Zielsequenz im Schritt c) kann mittels einer beliebigen zeitaufgelösten Fluoreszenz-Spektroskopie erfolgen, die in der Lage ist, in einem sehr kleinen Volumenelement sehr geringe Fluoreszenzsignale bis hinunter zur Einzelphotonenzählung zu erfassen. Wichtig ist dabei, daß durch die Zeitauflösung die von freien Primern stammenden Signale sich deutlich von denen unterscheiden, die von durch die Zielsequenz sozusagen aufkonzentrierten Primern stammen.

Vorzugsweise erfolgt dies mittels einer Korrelationsfluoreszenz-Spektroskopie, bei der ein sehr kleines Volumenelement, vorzugsweise 0,1 - 20 fl der inkubierten Lösung einem Anregungslicht eines Laser ausgesetzt wird, das die in diesem Meßvolumen befindlichen Primer zur Emission von Fluoreszenzlicht anregt, wobei das emittierte Fluoreszenzlicht aus dem Meßvolumen mittels eines Photodetektors gemessen wird, und eine Korrelation zwischen der zeitlichen Veränderung der gemessenen Emission und der relativen Diffusionsgeschwindigkeit der beteiligten Moleküle erstellt wird, so daß bei entsprechend starker Verdünnung einzelne Moleküle in dem Meßvolumen identifiziert werden. Dabei ist es dann bevorzugt, wenn ein elektrisches Feld verwendet wird, um die Driftgeschwindigkeit der mit Primern hybridisierten Zielsequenzen über die Diffusionsgeschwindigkeit hinaus zu erhöhen.

Da ein großes RNA- oder DNA-Molekül relativ langsam diffundiert, muß man es nämlich mit Hilfe spezieller Verfahren in der Lichtfalle aufkonzentrieren. Im Falle gewöhnlicher Diffusion, unter Annahme eines Diffusionskoeffizienten von $5 \times 10^{-8} \mathrm{cm}^2/\mathrm{s}$ wie er für M13-Phagen gemessen wurde, beträgt die mittlere Zeit für die Diffusion einer Zielsequenz in eine Lichtfalle vom Durchmesser 0,5 $\mu$m etwa $10^{-10}c^{-1}$ Sekunden, wobei c die molare Konzentration der Zielsequenz ist. Mit $c = 10^{-15}$ M ergäbe sich bereits eine Diffusionszeit von einem Tag.

Eine Verkürzung der Diffusionszeit läßt sich zwar auch durch die Vergrößerung des Raumelementes der Lichtfalle erreichen, hier sind jedoch enge Grenzen gesetzt, weil der Rauschhintergrund mit der dritten Potenz des Radius des Raumelementes ansteigt.

Eine wirksamere Methode ist die erwähnte Erhöhung der Driftgeschwindigkeit mit Hilfe elektrischer Felder, wodurch in vorteilhafter Weise die Meßzeit deutlich verkürzt werden kann.

Dabei ist es bevorzugt, wenn eine kapillarelektrophoretische Trennung von ungebundenen Primern und mit Primern hybridisierten Zielsequenzen erfolgt, wobei eine Kapillare mit einer Spitzenöffnung von kleiner 0,01 mm vor das Meßvolumen plaziert und in der Kapillare ein elektrisches Gleichfeld erzeugt wird, das die mit Primern hybridisierten, negativ geladenen Zielsequenzen in Richtung auf das Meßvolumen bewegt.

In vorteilhafter Weise kann hier eine ausgezogene Kapillare, eine sogenannte Neher-Kapillare verwendet werden, wie sie z.B. beschrieben ist in dem Artikel "The Patch Clamp Technique", E. Neher und B. Sakmann, in Scientific American, März 1992, Seiten 44 - 51. Aus der eingangs erwähnten WO 94/16313 ist es bereits bekannt, in einer derartigen Kapillare ein Feld von etwa 1 kV/cm zu erzeugen, was eine Wandungsgeschwindigkeit von ca. 1 mm/s bewirkt.

Anstelle der Kapillare kann auch eine Quadrupol- oder Radial-Ionenfalle eingesetzt werden, wobei letztere besonders wirksam ist. Sie besteht aus einer "punktförmigen" Spitze (z.B. Neher-Kapillare), die von einer Ringelektrode mit einem Durchmesser von bspw. 1 cm umgeben ist. Das Feld ist in diesem Falle ein Gleichfeld, mit positiv geladener Spitze der Kapillare. Die negativ geladene Zielstruktur reichert sich im inhomogenen Feld an der Spitze an. Dort gibt es ein Gleichgewicht zwischen Antransport durch Wanderung im Feld und Abtransport durch Diffusion. Die langsam diffundierenden Zielstrukturen reichern sich bis zu $10^3$-fach stärker an als ihre niedermolekularen Primer.

Andererseits ist es bevorzugt, wenn in Schritt c) ein hochfrequentes, inhomogenes elektrisches Wechselfeld verwendet wird, das in den Zielsequenzen ein Dipolmoment induziert, das als solches in dem inhomogenen Feld wandert.

Hier wird auf vorteilhafte Weise ein wesentlicher Nachteil der kapillarelektrophoretischen Trennung oder der Radial-Ionenfalle beseitigt, da eine Mitanreicherung nicht-markierter Primer vermieden wird.

In dem Artikel "Structure and kinetic properties of polyelectrolytes in solution, determined from relaxation phenomena in electric fields", M. Eigen und G. Schwarz, in "Electrolytes", Pergamon Press 1962, wurde gezeigt, daß stäbchenförmige Polyelektrolyte und insbesondere Nucleinsäuren eine elektrische Polarisierbarkeit besitzen, die aus einer Verschiebung der Ionenwolke relativ zum Poly-Ion im elektrischen Feld resultiert. Speziell bei DNA ergeben sich sehr große induzierte Dipolmomente, die bereits bei Feldstärken von $10^3$ Volt/cm zur vollständigen Ausrichtung im Feld führen. Die Relaxationszeit der Polarisierung liegt bei einer Mikrosekunde, so daß mit hochfrequenten Wechselfeldern bei etwa $10^5$ bis $10^6$ Hz gearbeitet werden kann. Die Ausrichtung erfolgt innerhalb von Millisekunden bis Sekunden.

Mit anderen Worten, hochfrequente Wechselfelder induzieren in Nucleinsäuren starke Dipolmomente, was durch eine Änderung der Leitfähigkeit einer Lösung mit derart beaufschlagten Nucleinsäuren gezeigt werden kann. Das bedeutet nun aber, daß Nucleinsäuren in einem inhomogenen hochfrequente Wechselfeld in Richtung der höheren Feldstärke wandern, was erfindungsgemäß ausgenutzt wird, um die markierten Zielsequenzen im Meßvolumen aufzukonzentrieren, da ein Dipol im inhomogenen Feld in Richtung der größten Feldstärke wandert, die so gelegt werden kann, daß sie sich im Meßvolumen befindet.

Dazu wird eine radiale Elektrodenanordnung verwendet, die aus einer in der Lichtfalle angebrachten Elektrodenspitze und einer ringförmigen Gegenelektrode besteht.

Weitere Vorteile ergeben sich aus der Beschreibung und der beigefügten Zeichnung.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:

Fig. 1    eine vereinfachte, schematische Darstellung einer Inkubationslösung aus Zielsequenz und Primern; und

Fig. 2    eine vereinfachte, schematische Darstellung einer Apparatur für optische Analysen mit einer nicht maßstabsgetreu eingezeichneten elektrische Molekülfalle, die ein hochfrequentes, inhomogenes elektrisches Wechselfeld verwendet.

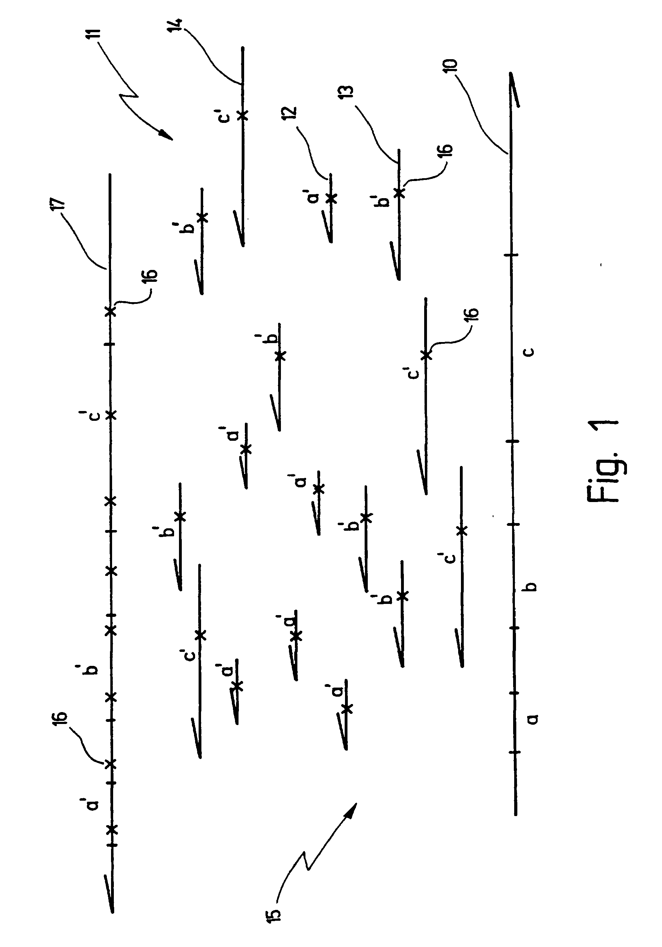

In Fig. 1 ist schematisch ein Nucleinsäurestrang in Form einer Zielsequenz 10 dargestellt, bei der einzelne, ausgewählte Sequenzabschnitte mit a, b und c bezeichnet sind.

Neben der Zielsequenz 10 ist ein Gemisch 11 von Primern 12, 13 und 14 dargestellt, die sich zusammen mit der Zielsequenz 10 in einer Inkubationslösung 15 befinden.

Die Primer oder Oligonucleotide 12 weisen eine zu dem Abschnitt a komplementäre Sequenz a' auf, während die Primer 13 und 14 komplementäre Sequenzen zu den Abschnitten b bzw. c aufweisen.

Die Primer 12, 13 und 14 sind jeweils mit einem oder mit mehreren gleichen Farbstoffmolekülen 16 markiert, die bei entsprechender Anregung ein Fluoreszenzlicht aussenden. Die Farbstoffmoleküle 16 sind in Fig. 1 durch ein Kreuz symbolisiert.

Statt vieler Primer 12, 13, 14 kann auch ein mehr oder weniger kompletter Antisense-Strang 17 eingesetzt oder durch Polymerisation der Zielsequenz 10 zu einem Doppelstrang erzeugt werden, der mit vielen Farbstoffmolekülen 16 markiert und zu der ganzen Zielsequenz 10 komplementär ist.

Wenn die Lösung 15 bei einer Temperatur inkubiert wird, die so hoch ist, daß mögliche Sekundärstrukturen der Zielsequenz 10 aufschmelzen, aber hinreichend tief ist, daß eine spezifische Bindung zwischen den Primern 12, 13 und 14 und den entsprechenden Abschnitten a, b und c der Zielsequenz 10 nicht aufschmelzen, dann lagern sich die Primer 12, 13, 14 an der Zielsequenz 10 an. Die Inkubationstemperatur muß dabei hinreichend hoch sein, so daß unspezifische Bindungen zwischen Primern 12, 13 und 14 und der Zielsequenz 10 wieder aufschmelzen.

Auf diese Weise erhält man eine Lösung 15, in der die Zielsequenz 10 oder die wenigen Zielsequenzen 10 jeweils mehrere Primer 12, 13, 14 tragen, wobei die Primer eine Sequenzlänge zwischen 15 und 20 Bausteinen aufweisen und insgesamt 20 bis 100 verschiedene Primer 12, 13, 14 bereitgestellt sind.

Die Primer 12, 13 und 14 können entweder durch direkte Synthese bspw. mittels eines Nucleinsäure-Synthetisierers hergestellt werden, wobei z.B. mittels eines DNA-Synthetisierers kurze farbstoffmarkierte DNA-Stränge hergestellt werden, die Abschnitte a, b oder c der Zielsequenz 10 aufweisen. Diese DNA-Stränge werden dann bspw. mit der T7-Polymerase transkribiert und die Transkriptionsprodukte als Primer 12, 13, 14 verwendet. Dieses Herstellungsverfahren für das "Cocktail" genannte Gemisch 11 setzt voraus, daß die Abschnitte a, b und c in der Sequenz bekannt sind.

Soll dagegen ein in der Sequenz teilweise oder ganz unbekannter Nucleinsäurestrang 10 als Zielsequenz verwendet werden, so werden die Primer 12, 13 und 14 durch Replikation oder Transkription der Zielsequenz in Gegenwart von mit Farbstoffmolekülen markierten Nucleinsäure-Bausteinen hergestellt. Dies kann bspw. durch Verwendung der Polymerase-Kettenreaktionstechnik, eines analogen Verfahrens oder mit Hilfe einer DNA- bzw. RNA-Polymerase, wie z.B. der T7-Polymerase, einer reversen Transkriptase etc. erfolgen.

Die Replikations- oder Transkriptionsprodukte werden anschließend in Teilsequenzen zerschnitten, wobei entweder mechanische Verfahren oder spezifisch und/oder unspezifisch schneidende Nucleasen eingesetzt werden, um die Produkte zu Primern abzudauen. Hier muß selbstverständlich sichergestellt werden, daß in dem Gemisch 11 keine der ursprünglich als Vorlage für die Transkription/Replikation verwendeten Zielsequenzen mehr enthalten ist, um falsch-positive Anzeigen zu vermeiden.

Als Zielsequenz 10 und Primer 12, 13 und 14 können sowohl RNA-als auch DNA-Moleküle verwendet werden. Ferner ist es möglich, als Primer 12, 13, 14 sogenannte PNA-Moleküle zu verwenden, die mit positiver Ladung versehen werden können, so daß sich eine Abtrennung der freien Primer 12, 13, 14 von den Zielsequenzen 10 mit daran gebundenen Primern 12, 13, 14 auf elektrophoretischem Wege bewerkstelligen läßt.

Bei Verwendung des Antisense-Stranges 17 kann dieser entweder der Untersuchungslösung zugegeben werden, woraufhin dann ein entsprechendes Temperverfahren für die Doppelstrangbildung sorgt. Andererseits ist es auch möglich, der Zielsequenz 10 farbstoffmarkierte Nucleinsäure-Bausteine zuzugeben und mit Hilfe einer Polymerase die Zielsequenz 10 zu einem Doppelstrang aus Zielsequenz 10 und Antisense-Strang 17 aufzupolymerisieren. Die Spezifität bezüglich der nachzuweisenden Zielsequenz 10 wird dadurch erreicht, daß der Zielsequenz 10 zunächst ein spezifisches Oligonucleotid zugegeben werden muß, das mit dem Stranganfang der Zielsequenz 10 eine doppelsträngige Region bildet, die dann als Primer für die Polymerase dient. Unterschiedliche Zielsequenzen 10 können so durch unterschiedliche Primer für die Polymerase ausgewählt werden. Auf diese Weise wird die Zielsequenz zu einem mit vielen Farbstoffmolekülen markierten Doppelstrang aufpolymerisiert.

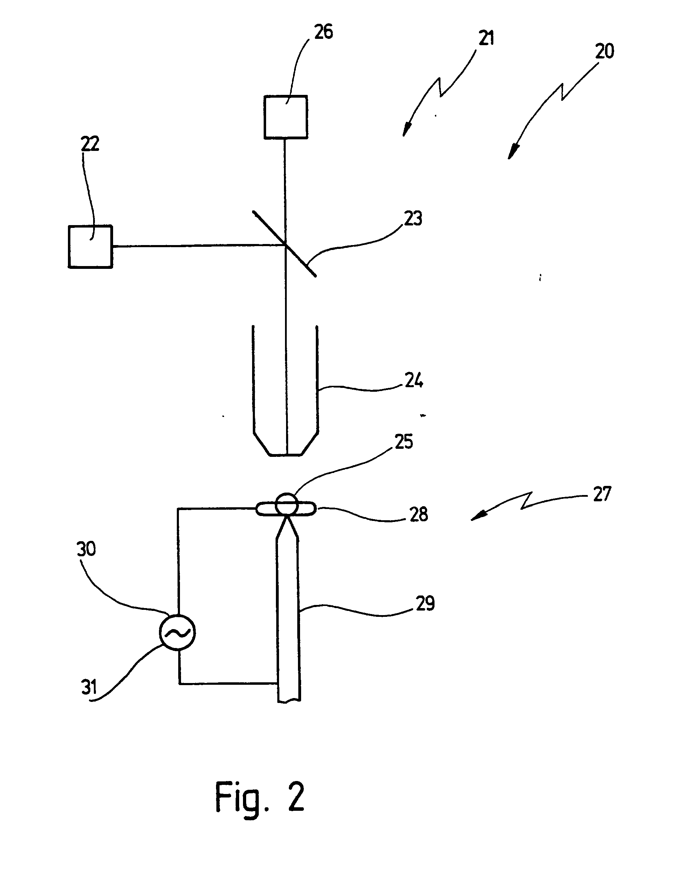

Ein Teil der Inkubationslösung 15 wird dann in eine in Fig. 2 schematisch dargestellte und mit 20 bezeichnete Apparatur für optische Analysen gegeben, die dazu ausgelegt ist, wenige, vorzugsweise einen der nachzuweisenden Nucleinsäurestränge, an die bzw. den ein oder mehrere Primer hybridisiert sind bzw. ist, vor dem Hintergrund der nicht hybridisierten Primer zu diskriminieren.

Die in Fig. 2 gezeigte Apparatur 20 ist eine FCS-Apparatur, wie sie bspw. aus der eingangs erwähnten WO 94/16313 bekannt ist. Für eine ausführliche Beschreibung des Aufbaus und der Funktion dieser FCS-Apparatur 21 wird auf diese Druckschrift sowie die darin aufgeführten Zitatstellen verwiesen.

In einem kurzen Überblick umfaßt die FCS-Apparatur 21 einen Laser 22, der über einen Strahlteiler 23 und eine konfokale

Abbildungsoptik 24 ein kleines Meßvolumen 25 ausleuchtet, das im Bereich zwischen 0,1 und 10 fl liegt. Dieses Meßvolumen 25 wird über die konfokale Optik 24 auf einen Photodetektor 26 abgebildet, der mit einer nicht weiter dargestellten Auswerteelektronik für die FCS-Analyse verbunden ist.

Das Meßvolumen 25 bedeckt einen kleinen Teil der Inkubationslösung 15, die in Fig. 2 aus Gründen der Übersichtlichkeit nicht dargestellt ist.

Beinhaltet die Inkubationslösung 15 den oben erwähnten, farbstoffmarkierten Doppelstrang, so müssen ggf. noch die freien farbstoffmarkierten Nucleinsäure-Bausteine ggf. elektrophoretisch abgetrennt werden, bevor die Inkubationslösung 15 in die Apparatur 20 gegeben werden kann. Wegen der vielen in dem Doppelstrang befindlichen Farbstoffmoleküle erscheint ein in das Meßvolumen 25 wandernder derartiger Doppelstrang als Lichtblitz und ist somit ohne weiteres identifizierbar. Da der zugegebene oder erzeugte Antisense-Strang 17 nur spezifisch zu der Zielsequenz 10 "paßt", bedeutet ein derartiger Lichtblitz, daß die nachzuweisenden Nucleinsäurestränge tatsächlich die Zielsequenz 10 aufweisen.

Um die mit Primern 12, 13, 14 hybridisierten Zielsequenzen 10 schneller als diffusionskontrolliert in das Meßvolumen 25 zu befördern, ist in Fig. 2 eine elektrische Molekülfalle 27 vorgesehen, die in dem gezeigten Ausführungsbeispiel eine Ringelektrode 28 sowie eine Neher-Kapillare 29 umfaßt, deren ausgezogene Spitze zentrisch zu der Ringelektrode 28 liegt.

Wird jetzt zwischen Ringelektrode 28 und Neher-Kapillare 29 eine elektrische Spannung angelegt, so erfolgt eine Drift der Zielsequenzen 10 in das Meßvolumen 25. Wenn die Primer 12, 13, 14 und die Zielsequenzen 10 unterschiedliche Ladungen aufweisen, erhält man gleichzeitig eine elektrophoretische Trennung.

In dem gezeigten Ausführungsbeispiel ist die Spannung 30 jedoch eine Wechselspannung 31, die zwischen der Ringelektrode 28 und der Neher-Kapillare 29 ein inhomogenes, hochfrequentes elektrisches Wechselfeld erzeugt. Die Frequenz dieses Wechselfeldes liegt dabei vorzugsweise im Bereich von einigen 100 kHz.

Durch dieses elektrische Wechselfeld ergeben sich insbesondere bei DNA sehr große induzierte Dipolmomente, die bereits bei Feldstärken von $10^3$ Volt/cm zur vollständigen Ausrichtung im Feld führen. Die Relaxationszeit der Polarisierung liegt bei einer Mikrosekunde. Man kann daher mit hochfrequenten Wechselfeldern bei etwa $10^5$ bis $10^6$ Hz arbeiten, wobei die Ausrichtung in Millisekunden bis Sekunden erfolgt. Dieser Effekt wird nun ausgenutzt, um die markierte Zielsequenz im Meßvolumen 25 zu konzentrieren.

Die elektrische Molekülfalle 27 verwendet dabei die in dem Meßvolumen 25 angeordnete Elektrodenspitze der Neher-Kapillare 29, wobei die Elektrodenspitze einen Durchmesser von 1 $\mu$m aufweist. Die ringförmige Gegenelektrode 25 hat dagegen einen Durchmesser von 1 cm.

Die in Fig. 2 der Übersichtlichkeit wegen nicht gezeigte Inkubationslösung 15 füllt den Raum zwischen den beiden Elektroden 29, 28 aus. In diesem Raum entsteht bei Anlegen der Spannung 30, 31 ein inhomogenes Radialfeld, dessen Feldstärke proportional zu 1/r ist. Bei einer Elektrodenspannung von 10 Volt erreicht man daher an der Spitze bei r = 1 $\mu$m eine Feldstärke von $10^5$ Volt/cm.

Der in der Zielsequenz 10 induzierte Dipol wird in diesem inhomogenen Feld in den Bereich der höchsten Feldstärke, also in den Bereich des Meßvolumens 25 gezogen.

Da das erreichbare Dipolmoment proportional zum Quadrat der Länge ist, wobei im Falle von DNA die Äquivalenzlänge angesetzt wird, lassen sich Feldstärken wählen, auf die nur lange, markierte Nucleinsäurestränge, nicht aber kurzkettige Primersequenzen ansprechen. Da ein hochfrequentes Wechselfeld angelegt wird, sprechen auch große starre Dipole wie z.B. Proteinmoleküle auf die elektrische Falle 27 nicht an.

Auf diese Weise ist es also möglich, durch Verwendung des Cocktails die markierten Primersequenzen 12, 13, 14 an der Zielsequenz 10 anzureichern und diese Zielsequenz 10 mit anhybridisierten Primern 12, 13, 14 in das Meßvolumen 25 der Apparatur 20 wandern zu lassen. Die Verwendung des Cocktails erhöht dabei die Intensität des gemessenen Signales, während die Verwendung der elektrischen Molekülfalle 27 die erforderliche Meßzeit deutlich reduziert, da der Diffusions- eine Driftgeschwindigkeit überlagert wird.

Mit dem neuen Verfahren, dem neuen Gemisch 11 sowie der neuen Vorrichtung 20 ist es damit möglich, einzelne Nucleinsäurestränge bekannter und/oder unbekannter Sequenz innerhalb akzeptabler Meßzeiten nachzuweisen.

Wie die nachfolgende Überlegung zeigt, sind die im Meßvolumen 25 auftretenden Wärmeeffekte vernachlässigbar:

Die dissipierte Energie bei einer elektrischen Leitfähigkeit σ und einer elektrischen Feldstärke E beträgt im homogenen Fall einer Kapillare der Länge l und vom Durchmesser d:

$$Q_{el} = \sigma E^2 l d^2 \pi / 4$$

Das elektrische Wärmeäquivalent beträgt dabei ungefähr 0,2 cal/Ws. Um $Q_{el}$ durch Wärmeableitung aus der Flüssigkeit zu entfernen, bedarf eines stationären Temperaturgradienten dT/dr, wobei angenommen wird, daß das Medium eine Wärmeleitfähigkeit λ aufweist:

$$0,2 \sigma E^2 l d^2 \pi / 4 = \lambda l d \pi \frac{dT}{dr}$$

oder:

$$\frac{dT}{dr} = \frac{\sigma E^2}{\lambda \cdot 20} d$$

In einem typischen Beispiel, bei dem λ für Wasser mit $10^{-3}$ cal/(cm grad sec) angenommen wird, wobei $\sigma < 10^{-3}$ Siemens und E = 10 Volt/cm ist, ergibt sich ein Temperaturgradient von kleiner 0,5 grad/cm, wenn die Kapillare einen Durchmesser von 0,1 cm aufweist. Unter der Annahme, daß der hauptsächliche Wärmestau in Wasser und nicht in der Kapillarenwandung erfolgt, sollte sich damit ein Serum ohne übermäßig starke Verdünnung direkt untersuchen lassen.

Ähnliche Betrachtungen ließen sich auch für die radiale Molekülfalle 27 anstellen. Im Bereich der Elektrodenspitze der Neher-Kapillare 29 beträgt die Feldstärke zwar $10^5$ Volt/cm, so daß im Bereich eines μm ein Temperaturgradient von $10^5$ grad/cm entsteht, dies entspricht jedoch einer Überhitzung von 10 Grad im Bereich eines Mikrometers.

Abschließend sei noch erwähnt, daß die Empfindlichkeit des Meßverfahrens weiter gesteigert werden kann, indem zwei oder mehrere Fluoreszenzfarbstoffe verwendet und eine Kreuzkorrelation durchgeführt wird, wie es aus der mehrfach erwähnten WO 94/16313 bereits in allgemeiner Form bekannt ist.

**Patentansprüche**

1. Verfahren zum direkten Nachweisen weniger, vorzugsweise einzelner Nucleinsäurestränge einer bestimmten Zielsequenz (10) in einer Untersuchungslösung, mit den Schritten:

    a) Bereitstellen einer Testlösung mit einem Gemisch (11) unterschiedlicher, kurzer Primer (12, 13, 14), die jeweils eine zu einem Abschnitt (a, b, c) der Zielsequenz (10) komplementäre, sogenannte Antisense-Sequenz (a', b', c') aufweisen und mit einem oder mehreren gleichen Farbstoffmolekülen (16) markiert sind,
    b) Mischen der Testlösung mit der Untersuchungslösung und Inkubieren dieser gemischten Lösung (15) unter Hybridisierung der Primer (12, 13, 14) mit den nachzuweisenden Nucleinsäuresträngen zulassenden Randbedingungen, und dann
    c) Identifizieren der Zielsequenz (10) in der inkubierten Lösung (15) mit einer Apparatur (20) für optische Analysen mittels zeitaufgelöster Fluoreszenzspektroskopie, die dazu ausgelegt ist, wenige, vorzugsweise einen der nachzuweisenden Nucleinsäurestränge, an die bzw. den mehrere Primer (12, 13, 14) hybridisiert sind, vor dem Hintergrund der nicht hybridisierten Primer (12, 13, 14) zu diskriminieren.

2. Verfahren nach Anspruch 1, **dadurch gekennzeichnet, daß** in Schritt a) zu unterschiedlichen, vorzugsweise nicht überlappenden Abschnitten (a, b, c) der Zielsequenz (10) komplementäre Primersequenzen (12, 13, 14) bereitgestellt werden, wobei unterschiedliche Primersequenzen (12, 13, 14) sich in der Sequenzabfolge der Nucleinsäure-(oder analoger) Bausteine voneinander unterscheiden.

3. Verfahren nach Anspruch 1 oder 2, **dadurch gekennzeichnet, daß** in Schritt a) Primer (12, 13, 14) unterschiedlicher Sequenzlänge, also mit unterschiedlicher Zahl der Nucleinsäure-(oder analoger) Bausteine pro Primer (12, 13, 14) bereitgestellt werden.

4. Verfahren nach einem der Ansprüche 1 bis 3, **dadurch gekennzeichnet, daß** in Schritt a) Primer (12, 13, 14) mit einer Sequenzlänge zwischen 10 und 50, vorzugsweise 15 und 20 Nucleinsäure-(oder analogen) Bausteinen je Primer (12, 13, 14) bereitgestellt werden.

5. Verfahren nach einem der Ansprüche 1 bis 4, **dadurch gekennzeichnet, daß** in Schritt a) Primer (12, 13, 14) mit 10 bis 200, vorzugsweise 20 bis 100 verschiedenen Sequenzen bereitgestellt werden.

6. Verfahren nach einem der Ansprüche 1 bis 5, **dadurch gekennzeichnet, daß** die Primer (12, 13, 14) für Schritt a) durch direkte Synthese, bspw. mittels eines Nucleinsäure-Synthetisierers, hergestellt werden.

7. Verfahren nach einem der Ansprüche 1 bis 5, **dadurch gekennzeichnet, daß** die Primer (12, 13, 14) für Schritt a) durch Replikation oder Transkription der Zielsequenz in Gegenwart von mit Farbstoffmolekülen markierten Nucleinsäure- (oder analogen) Bausteinen hergestellt werden, wobei die Replikations- oder Transkriptionsprodukte anschließend in Teilsequenzen zerschnitten werden, die dann als Primer (12, 13, 14) verwendet werden.

8. Verfahren nach Anspruch 7, **dadurch gekennzeichnet, daß** die Replikations- oder Transkriptionsprodukte für bestimmte Zeitspannen der Wirkung spezifisch und/oder unspezifisch schneidender Nucleasen ausgesetzt werden, um sie zu Primern (12, 13, 14) abzudauen.

9. Verfahren nach Anspruch 7 oder 8, **dadurch gekennzeichnet, daß** die Replikations- oder Transkriptionsprodukte durch Verwendung der Polymerase-Rettereraktionstechnik (PCR-Technik), eines analogen Verfahrens oder mit Hilfe anderer spezifischer RNA-bzw. DNA-Polymerasen hergestellt werden.

10. Verfahren nach einem der Ansprüche 1 bis 6, **dadurch gekennzeichnet, daß** mittels eines DNA-Synthesizers kurze DNA-Stränge hergestellt werden, die Abschnitte der Zielsequenz aufweisen, daß diese DNA-Stränge bspw. mit der T 7-Polymerase transkribiert und die Transkriptionsprodukte als Primer (12, 13, 14) verwendet werden.

11. Verfahren nach einem der Ansprüche 1 bis 10, **dadurch gekennzeichnet, daß** als Primer (12, 13, 14) DNA-, RNA- oder PNA-Sequenzen verwendet werden.

12. Verfahren nach einem der Ansprüche 1 bis 11, **dadurch gekennzeichnet, daß** in Schritt b) die gemischte Lösung (12) bei einer Temperatur inkubiert wird, die so hoch ist, daß Tertiär- und Sekundärstrukturen der Zielsequenz (10) aufschmelzen, die hinreichend tief ist, so daß die spezifischen Bindungen zwischen Primer (12, 13, 14) und Zielsequenz (10) nicht aufschmelzen, und die hinreichend hoch ist, so daß unspezifische Bindungen zwischen Primer (12, 13, 14) und Zielsequenz (10) aufschmelzen.

13. Verfahren nach einem der Ansprüche 1 bis 12, **dadurch gekennzeichnet, daß** in Schritt b) die Primer (12, 13, 14) in großem Überschuß gegenüber der Zielsequenz (10) eingesetzt werden.

14. Verfahren nach Anspruch 13, **dadurch gekennzeichnet, daß** nach erfolgter Inkubation die ungebundenen Primer (12, 13, 14) von der hybridisierten Zielsequenz (10) abgetrennt werden.

15. Verfahren nach Anspruch 14, **dadurch gekennzeichnet, daß** als Primer (12, 13, 14) positiv geladene PNA-Sequenzen verwendet werden, und die Abtrennung auf elektrophoretischem Wege erfolgt.

16. Verfahren nach einem der Ansprüche 1 bis 15, **dadurch gekennzeichnet, daß** das Identifizieren der Zielsequenz (10) in Schritt c) mittels einer Korrelationsfluoreszenz-Spektroskopie erfolgt, bei der ein sehr kleines Volumenelement (25), vorzugsweise 0,1 - 20 fl der inkubierten Lösung einem Anregungslicht eines Lasers (22) ausgesetzt wird, das die in diesem Meßvolumen (25) befindlichen Primer (12, 13, 14) zur Emission von Fluoreszenzlicht anregt, das emittierte Fluoreszenzlicht aus dem Meßvolumen (25) mittels eines Photodetektors (26) gemessen wird, und eine Korrelation zwischen der zeitlichen veränderung der gemessenen Emission und der relativen Diffusionsgeschwindigkeit der beteiligten Moleküle erstellt wird, so daß bei entsprechend starker Verdünnung einzelne Moleküle in dem Meßvolumen (25) identifiziert werden.

17. Verfahren nach Anspruch 16, **dadurch gekennzeichnet, daß** in Schritt c) ein elektrisches Feld verwendet wird, um die Driftgeschwindigkeit der mit Primern (12, 13, 14) hybridisierten Zielsequenzen (10) über die Diffusionsgeschwindigkeit hinaus zu erhöhen.

18. Verfahren nach Anspruch 17, **dadurch gekennzeichnet, daß** in Schritt c) eine kapillarelektrophoretische Trennung von ungebundenen Primern (12, 13, 14) und mit Primern (12, 13, 14) hybridisierten Zielsequenzen (10) erfolgt, wobei eine Kapillare (29) mit einer Spitzenöffnung von kleiner 0,01 mm vor das Meßvolumen (25) plaziert und in der Kapillare (29) ein elektrisches Gleichfeld erzeugt wird, das die mit Primern (12, 13, 14) hybridisierten, negativ geladenen Zielsequenzen (10) in Richtung auf das Meßvolumen (25) bewegt.

19. Verfahren nach Anspruch 17, **dadurch gekennzeichnet, daß** eine Quadrupol- oder Radial-Ionenfalle verwendet wird, bei der eine Spitze einer Kapillare (29) von einer Ringelektrode (28) umgeben ist, die ein inhomogenes Feld erzeugt, in dessen maximalem Feldstärkebereich das Meßvolumen (25) liegt.

20. Verfahren nach Anspruch 17 oder 19, **dadurch gekennzeichnet, daß** in Schritt c) ein hochfrequentes inhomogenes elektrisches Wechselfeld verwendet wird, das in den Zielsequenzen (10) ein Dipolmoment induziert, das als solches in dem inhomogenen Feld wandert.

21. Verfahren nach Anspruch 20, **dadurch gekennzeichnet, daß** das Wechselfeld eine Frequenz von mehreren 100 kHz aufweist.

22. Verfahren zum direkten Nachweisen weniger, vorzugsweise einzelner Nucleinsäurestränge einer bestimmten Zielsequenz (10) in einer Untersuchungslösung, mit den Schritten:

1) Hybridisieren zumindest einiger der nachzuweisenden Nucleinsäurestränge mit jeweils einem langen Antisense-Strang (17), der mit mehreren gleichen Farbstoffmolekülen (16) markiert und zu der gesamten oder nahezu der gesamten Zielsequenz (10) komplementär ist, und
2) Identifizieren der Zielsequenz (10) mit einer Apparatur (20) für optische Analysen mittels zeitaufgelöster Fluoreszenzspektroskopie, die dazu ausgelegt ist, wenige, vorzugsweise einen der nachzuweisenden Nucleinsäurestränge, an die bzw. den ein Antisense-Strang (17) hybridisiert ist, zu diskriminieren.

23. Verfahren nach Anspruch 22, **dadurch gekennzeichnet, daß** in Schritt 1) die Hybridisierung zwischen nachzuweisendem Nucleinsäurestrang und Antisense-Strang (17) bei Einsatz von farbstoffmarkierten Nucleinsäure-(oder analogen) Bausteinen durch direktes Aufpolymerisieren des nachzuweisenden Nucleinsäurestranges zu einem Doppelstrang aus nachzuweisendem Nucleinsäurestrang und Antisense-Strang (17) erfolgt.

**Claims**

1. A process for the direct detection of a few, preferably singular, nucleic acid strands of a particular target sequence (10) in a sample solution, comprising the steps of:

a) providing a test solution with a mixture (11) of different short primers (12, 13, 14) each having a so-called antisense sequence (a', b', c') complementary to a section (a, b, c) of the target sequence (10) and being marked with one or more identical dye molecules (16);
b) mixing said test solution with the sample solution and incubating this mixed solution (15) under conditions allowing the hybridization of the primers (12, 13, 14) with the nucleic acid strands to be detected, and then
c) identifying the target sequence (10) in the incubated solution (15) using an apparatus (20) for optical analyses by means of time-resolved fluorescence spectroscopy adapted for discrim

inating a few, preferably one, of the nucleic acid strands to be detected to which several primers (12, 13, 14) are hybridized against the background of the non-hybridized primers (12, 13, 14).

2. The process according to claim 1, **characterized in that** primer sequences (12, 13, 14) complementary to different, preferably non-overlapping, sections (a, b, c) of the target sequence (10) are provided in step a), wherein different primer sequences (12, 13, 14) differ from one another in the sequence of their nucleic acid (or analogous) moieties.

3. The process according to claim 1 or 2, **characterized in that** primers (12, 13, 14) of different sequence lengths, i.e., with different numbers of nucleic acid (or analogous) moieties per primer (12, 13, 14) are provided in step a).

4. The process according to any of claims 1 to 3, **characterized in that** primers (12, 13, 14) with a sequence length of from 10 to 50, preferably from 15 to 20, nucleic acid (or analogous) moieties per primer (12, 13, 14) are provided in step a).

5. The process according to any of claims 1 to 4, **characterized in that** primers (12, 13, 14) with from 10 to 200, preferably from 20 to 100, different sequences are provided in step a).

6. The process according to any of claims 1 to 5, **characterized in that** the primers (12, 13, 14) for step a) are prepared by direct synthesis or by means of a nucleic acid synthesizer.

7. The process according to any of claims 1 to 5, **characterized in that** the primers (12, 13, 14) for step a) are prepared by replication or transcription of the target sequence in the presence of nucleic acid (or analogous) moieties marked with dye molecules, wherein the replication or transcription products are subsequently cut into partial sequences which are then used as primers (12, 13, 14).

8. The process according to claim 7, **characterized in that** the replication or transcription products are exposed to the effect of specifically and/or non-specifically cutting nucleases for certain periods of time to digest them to primers (12, 13, 14).

9. The process according to claim 7 or 8, **characterized in that** the replication or transcription products are prepared by using polymerase chain reaction technique (PCR technique), an analogous method or by means of other specific RNA or DNA polymerases.

**10.** The process according to any of claims 1 to 6, **characterized in that** short DNA strands including sections of the target sequence are prepared by means of a DNA synthesizer, said DNA strands are transcribed, for example, with T7 polymerase, and the transcription products are used as primers (12, 13, 14).

**11.** The process according to any of claims 1 to 10, **characterized in that** DNA, RNA or PNA sequences are used as primers (12, 13, 14).

**12.** The process according to any of claims 1 to 11, **characterized in that** the incubation of the mixed solution in step b) is effected at a temperature which is high enough that tertiary and secondary structures in the target sequence (10) will melt, and low enough that the specific binding between primers (12, 13, 14) and the target sequence (10) will not melt, and high enough so that non-specific binding between primers (12, 13, 14) and the target sequence (10) will melt.

**13.** The process according to any of claims 1 to 12, **characterized in that** the primers (12, 13, 14) are employed in a large excess over the target sequence (10) in step b).

**14.** The process according to claim 13, **characterized in that** the free primers (12, 13, 14) are separated from the hybridized target sequence (10) after the incubation.

**15.** The process according to claim 14, **characterized in that** positively charged PNA sequences are used as primers (12, 13, 14), and the separation is carried out electrophoretically.

**16.** The process according to any of claims 1 to 15, **characterized in that** the identification of the target sequence (10) in step c) is carried out by means of fluorescence correlation spectroscopy wherein a very small volume element (25), preferably from 0.1 to 20 femtoliters, of the incubated solution is exposed to an excitation light from a laser (22) which excites the primers (12, 13, 14) present in this measuring volume (25) to emit fluorescence light, the fluorescence light emitted from the measuring volume is measured by means of a photodetector (26), and a correlation is drawn between the temporal change in the measured emission and the relative rate of diffusion of the molecules involved, so that with a correspondingly high dilution, individual molecules are identified in the measuring volume (25).

**17.** The process according to claim 16, **characterized in that** an electric field is used in step c) to increase the drift speed of the target sequences hybridized with primers (12, 13, 14) beyond the rate of diffusion.

**18.** The process according to claim 17, **characterized in that** a capillary electrophoretic separation of free primers (12, 13, 14) and target sequences (10) hybridized with primers (12, 13, 14) is carried out in step c), wherein a capillary (29) with a tip opening of less than 0.01 mm is placed in front of the measuring volume (25) and an electric DC field is generated in the capillary (29) which moves the negatively charged target sequences (10) hybridized with primers (12, 13, 14) towards the measuring volume (25).

**19.** The process according to claim 17, **characterized in that** a quadrupole or radial ion trap is used in which a tip of a capillary (29) is surrounded by an annular electrode (28) which generates an inhomogeneous field whose maximum field strength zone includes the measuring volume (25).

**20.** The process according to claim 17 or 19, **characterized in that** a highfrequency, inhomogeneous electric alternating field is used in step c) which induces a dipole moment in the target sequences (10) that, being a dipole moment, migrates in the inhomogeneous field.

**21.** The process according to claim 20, **characterized in that** the alternating field has a frequency of several 100 kHz.

**22.** A process for the direct detection of a few, preferably singular, nucleic acid strands of a particular target sequence (10) in a sample solution, comprising the steps of:

1) hybridizing each of at least some of the nucleic acid strands to be detected with one long antisense strand (17) that is labeled with several identical dye molecules (16) and complementary to the whole or almost the whole target sequence (10); and
2) identifying the target sequence (10) using an apparatus (20) for optical analyses by means of time-resolved fluorescence spectroscopy adapted for discriminating a few, preferably one, of the nucleic acid strands to be detected to which an antisense strand (17) is hybridized.

**23.** The process according to claim 22, **characterized in that** said hybridization between the nucleic acid strand to be detected and the antisense strand (17) in step 1) is effected by directly polymerizing the nucleic acid strand to be detected to form a double strand consisting of the nucleic acid strand to be detected and the antisense strand (17) when dye-labeled nucleic acid (or analogous) moieties are used.

**Revendications**

1. Procédé de détection directe de brins d'acide nucléique en petit nombre, de préférence de brins d'acide nucléique individuels, d'une séquence cible déterminée (10) dans une solution d'essai, comprenant les étapes consistant à :

a) fournir une solution test contenant un mélange (11) de différentes amorces courtes (12, 13, 14), qui présentent chacune une séquence appelée séquence anti-sens (a', b', c') complémentaire d'un segment (a, b, c) de la séquence cible (10) et qui sont marquées avec une ou plusieurs molécules de colorant identiques (16),
b) mélanger la solution test avec la solution d'essai et incuber cette solution mixte (15) pour que les amorces (12, 13, 14) s'hybrident sur les brins d'acide nucléique à détecter dans des conditions acceptables pour ceux-ci, puis
c) identifier la séquence cible (10) dans la solution incubée (15) à l'aide d'un appareil (20) pour analyses optiques au moyen d'une spectroscopie de fluorescence résolue dans le temps, lequel appareil est conçu pour discriminer, par rapport au bruit de fond des amorces non hybridées (12, 13, 14), un petit nombre, de préférence un seul, des brins d'acide nucléique à détecter sur lesquels ou lequel se sont hybridées plusieurs amorces (12, 13, 14).

2. Procédé selon la revendication 1, **caractérisé en ce que** des séquences d'amorçage (12, 13, 14) complémentaires de différents segments (a, b, c), de préférence non chevauchants, de la séquence cible (10) sont fournies à l'étape a), dans lequel les différentes séquences d'amorçage (12, 13, 14) diffèrent au niveau de la succession des éléments constitutifs de l'acide nucléique (ou analogues).

3. Procédé selon la revendication 1 ou 2, **caractérisé en ce que** des amorces (12, 13, 14) ayant différentes longueurs de séquence, à savoir ayant un nombre différent d'éléments constitutifs de l'acide nucléique (ou analogues) par amorce (12, 13, 14), sont fournies à l'étape a).

4. Procédé selon l'une quelconque des revendications 1 à 3, **caractérisé en ce que** des amorces (12, 13, 14) ayant une longueur de séquence comprise entre 10 et 50, de préférence entre 15 et 20 éléments constitutifs de l'acide nucléique (ou analogues) par amorce (12, 13, 14), sont fournies à l'étape a).

5. Procédé selon l'une quelconque des revendications 1 à 4, **caractérisé en ce que** des amorces (12, 13, 14) contenant 10 à 200, de préférence 20 à 100 séquences différentes, sont fournies à l'étape a).

6. Procédé selon l'une quelconque des revendications 1 à 5, **caractérisé en ce que** les amorces (12, 13, 14) pour l'étape a) sont préparées par synthèse directe, par exemple à l'aide d'un synthétiseur d'acide nucléique.

7. Procédé selon l'une quelconque des revendications 1 à 5, **caractérisé en ce que** les amorces (12, 13, 14) pour l'étape a) sont préparées par réplication ou transcription de la séquence cible en présence d'éléments constitutifs de l'acide nucléique (ou analogues) marqués avec des molécules de colorant, dans lequel les produits de réplication ou de transcription sont ensuite clivés en séquences partielles, qui sont ensuite utilisées en tant qu'amorces (12, 13, 14).

8. Procédé selon la revendication 7, **caractérisé en ce que** les produits de réplication ou de transcription sont soumis pendant une certaine durée à l'action de nucléases de restriction spécifiques et/ou non spécifiques pour être digérés et former des amorces (12, 13, 14).

9. Procédé selon la revendication 7 ou 8, **caractérisé en ce que** les produits de réplication ou de transcription sont préparés au moyen de la technique d'amplification en chaîne par polymérase (technique PCR), d'un procédé analogue ou à l'aide d'autres ADN- ou ARN-polymérases spécifiques.

10. Procédé selon l'une quelconque des revendications 1 à 6, **caractérisé en ce que** des brins d'ADN courts sont préparés à l'aide d'un synthétiseur d'ADN, lesquels brins présentent des segments de la séquence cible, **en ce que** ces brins d'ADN sont par exemple transcrits au moyen de la polymérase T7 et les produits de transcription sont utilisés en tant qu'amorces (12, 13, 14).

11. Procédé selon l'une quelconque des revendications 1 à 10, **caractérisé en ce que** des séquences d'ADN, d'ARN ou d'APN sont utilisées en tant qu'amorces (12, 13, 14).

12. Procédé selon l'une quelconque des revendications 1 à 11, **caractérisé en ce que**, à l'étape b), la solution mixte (12) est incubée à une température si élevée que les structures tertiaires et secondaires de la séquence cible (10) entrent en fusion, qui est suffisamment basse pour que les liaisons spécifiques entre l'amorce (12, 13, 14) et la séquence cible (10) n'entrent pas en fusion et qui est suffisamment haute pour que des liaisons non spécifiques entre l'amorce (12, 13, 14) et la séquence cible (10) entrent en fusion.

13. Procédé selon l'une quelconque des revendications 1 à 12, **caractérisé en ce que**, à l'étape b), les amorces (12, 13, 14) sont mises en oeuvre en large excès par rapport à la séquence cible (10).

**14.** Procédé selon la revendication 13, **caractérisé en ce que**, à l'issue de l'incubation, les amorces non liées (12, 13, 14) sont séparées de la séquence cible hybridée (10).

**15.** Procédé selon la revendication 14, **caractérisé en ce que** des séquences d'APN positivement chargées sont utilisées en tant qu'amorces (12, 13, 14) et la séparation est réalisée par voie électrophorétique.

**16.** Procédé selon l'une quelconque des revendications 1 à 15, **caractérisé en ce que** l'identification de la séquence cible (10) à l'étape c) est réalisée au moyen d'une spectroscopie à corrélation de fluorescence, dans laquelle un très petit élément de volume (25), de préférence de 0,1 à 20 fl, de la solution incubée est exposé à une lumière d'excitation d'un laser (22), qui excite les amorces (12, 13, 14) se trouvant dans ce volume de mesure (25) pour qu'elles émettent une lumière fluorescente, la lumière fluorescente émise par ce volume de mesure (25) est mesurée au moyen d'un photodétecteur (26), et une corrélation est établie entre la modification dans le temps de l'émission mesurée et la vitesse de diffusion relative des molécules impliquées, si bien qu'il est possible, à une forte dilution correspondante, d'identifier les molécules individuelles dans le volume de mesure (25).

**17.** Procédé selon la revendication 16, **caractérisé en ce que**, à l'étape c), un champ électrique est utilisé afin d'augmenter la vitesse de dérive des séquences cibles (10) hybridées avec les amorces (12, 13, 14) au-delà de la vitesse de diffusion.

**18.** Procédé selon la revendication 17, **caractérisé en ce que**, à l'étape c), une séparation par électrophorèse capillaire est réalisée entre les amorces non liées (12, 13, 14) et les séquences cibles (10) hybridées avec les amorces (12, 13, 14), dans laquelle un capillaire (29) présentant une ouverture de pointe inférieure à 0,01 mm est placé devant le volume de mesure (25) et un champ continu est généré dans le capillaire (29), lequel champ déplace les séquences cibles (10) chargées négativement et hybridées avec les amorces (12, 13, 14) en direction du volume de mesure (25).

**19.** Procédé selon la revendication 17, **caractérisé en ce qu'**un piège à ions quadripolaire ou radial est utilisé, dans lequel une pointe d'un capillaire (29) est entourée d'une électrode annulaire (28), qui génère un champ inhomogène, dans la zone d'intensité de champ maximale duquel se trouve le volume de mesure (25).

**20.** Procédé selon la revendication 17 ou 19, **caractérisé en ce que**, à l'étape c), un champ alternatif inhomogène à haute fréquence est utilisé, lequel champ induit un moment dipolaire dans les séquences cibles (10), lequel moment migre en tant que tel dans le champ inhomogène.

**21.** Procédé selon la revendication 20, **caractérisé en ce que** le champ alternatif présente une fréquence de plusieurs centaines de kHz.

**22.** Procédé de détection directe de brins d'acide nucléique en petit nombre, de préférence de brins d'acide nucléique individuels, d'une séquence cible déterminée (10) dans une solution d'essai, lequel procédé comprend les étapes consistant à :

1) hybrider au moins quelques-uns des brins d'acide nucléique à détecter à chaque fois sur un brin anti-sens long (17) marqué avec plusieurs molécules de colorant identiques (16) et complémentaire de l'ensemble, ou pratiquement de l'ensemble, de la séquence cible (10) et 2) identifier la séquence cible (10) à l'aide d'un appareil (20) pour analyses optiques au moyen d'une spectroscopie de fluorescence résolue dans le temps, lequel appareil est conçu pour discriminer un petit nombre, de préférence un seul des brins d'acide nucléique à détecter, sur lesquels ou lequel s'est hybridé un brin anti-sens (17).

**23.** Procédé selon la revendication 22, **caractérisé en ce que**, à l'étape 1), lors d'une utilisation d'éléments constitutifs de l'acide nucléique (ou analogues) marqués avec un colorant, l'hybridation du brin d'acide nucléique à détecter sur le brin anti-sens (17) a lieu par polymérisation directe du brin d'acide nucléique à détecter pour former un double brin constitué du brin d'acide nucléique à détecter et du brin anti-sens (17).

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9416313 A

**In der Beschreibung aufgeführte Nicht-Patentliteratur**

- **EIGEN H. et al.** *Proc. Natl. Acad. Sci. USA,* Juni 1994, vol. 91, 5740-5747
- **E. NEHER ; B. SAKMANN.** The Patch Clamp Technique. *Scientific American,* Marz 1992, 44-51
- Structure and kinetic properties of polyelectrolytes in solution, determined from relaxation phenomena in electric fields. **M. EIGEN ; G. SCHWARZ.** Electrolytes. Pergamon Press, 1962